(19)

Europäisches
Patentamt
European
Patent Office
Office européen
des brevets

(11)  **EP 4 552 575 A1**

(12)  **EUROPEAN PATENT APPLICATION**

(43) Date of publication:
**14.05.2025   Bulletin 2025/20**

(21) Application number: **23306923.6**

(22) Date of filing: **07.11.2023**

(51) International Patent Classification (IPC):
**A61B 5/375** (2021.01)    **A61B 5/00** (2006.01)
**A61B 5/16** (2006.01)    **G06F 3/01** (2006.01)

(52) Cooperative Patent Classification (CPC):
**A61B 5/4064; A61B 5/375; A61B 5/486;**
A61B 5/162; A61B 5/7267; A61B 2505/09;
G06F 3/015

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Université de Bourgogne
21000 Dijon (FR)**

• **INSTITUT NATIONAL DE LA SANTE ET DE LA
RECHERCHE MEDICALE - INSERM
75013 Paris (FR)**

(72) Inventor: **POZZO, Thierry
21000 Dijon (FR)**

(74) Representative: **Bandpay & Greuter
11 rue Christophe Colomb
75008 Paris (FR)**

(54)  **DEVICE FOR TRAINING MOTOR CORTICAL AREAS OF A BRAIN OF A HUMAN SUBJECT**

(57)     The invention notably relates to a device for training motor cortical areas of a brain of a human subject. The device comprises a display for providing the human subject with a set of visual stimuli, a speaker for providing the human subject with auditory stimuli, sensors for collecting responses of the human subject to the stimuli. A first sensor data collects a cognitive response of the human subject, a second sensor measures desynchronization of alpha waves of the brain, of low beta and Mu rhythm by using electroencephalogram techniques, a third sensor measures kinematics of at least one part of the body of the subject. From the collected responses, estimating by a processing unit a motor adaptation of the subject in response to one or more of the provided stimuli.

FIG. 15

Processed by Luminess, 75001 PARIS (FR)

**Description**

**FIELD OF THE INVENTION**

The invention relates to the field of computer programs and systems, and more specifically to a device for training motor cortical areas of a brain of a human subject.

**BACKGROUND**

Stroke-induced sensorimotor deficiencies are a major factor in dependency. For example, in France, the number of stroke victims is close to one million people, and around 150,000 people suffer a stroke every year (in 2014, 110.438 cases of hospitalization; in 2019, 123.000 cases of hospitalization). Worldwide, there are an estimated 15 million new cases each year, and 70 million new cases by 2030. Stroke-induced sensorimotor deficiencies affect an ever-growing population, around +15% per year. Strokes represent the leading cause of disability for patients who suffer severe after-effects. In addition, strokes are the second leading cause of intellectual decline after Alzheimer's and the third leading cause of death after cancer and cardiovascular disease. Finally, strokes are the leading cause of death among women. Stroke affects all age groups, and the average age of stroke patients is 73. For example, in the Paris region in 2010, there were 18,000 patients hospitalized for stroke, with almost 21,000 hospital stays per year and 3,000 stays in follow-up rehabilitation care. Children with cerebral palsy, who according to WHO estimates are diagnosed in 1.5 to 4 per thousand births, also present neuromotor difficulties.

Stroke rehabilitation methods have been developed. Five main types of stroke rehabilitation methods exist. The first one is conventional physical rehabilitation of the patient, where the patient actively or passively mobilizes their deficient limbs with the intervention of a therapist. This conventional solution requires the presence of medical staff, whose interactions with the patient are difficult to quantify. Moreover, the conventional physical rehabilitation is not possible when the patient is paralyzed or immobilized and should be best performed 1 to 2 weeks after the injury which is not always possible.

The second one is exoskeletons and robotic devices. They are expensive and require a specialized technical environment. Moreover, they can induce pain, and their mobilization does not exactly follow the kinematics of procedures They cannot be used while the patient is immobilized.

The third type of stroke rehabilitation method relies on virtual reality (VR) in the form of serious games. Training can involve either mastering a motivating artificial space or interacting with an avatar. However, the training is performed in an artificial sensory context that limits the transfer of learning to everyday gestures.

The fourth type is cognitive stimulation that involves stimulating explicit and reflective mental activity (e.g., crosswords, sudoku, mazes, etc.). The aim is not motor execution, but the conscious mental operation like mental arithmetic and logical-deductive tasks.

The fifth type of stroke rehabilitation method is the muscle electrostimulation. The electro simulator artificially produces muscle contractions in the absence of voluntary command by the patient. Electrostimulation reduces muscle loss due to the inactivity of isolated muscles, but in a non-ecological sensorimotor context.

The education and rehabilitation of the motor cortical areas of the human brain is not limited to the recovery of human brain damage caused by stroke. The training of the brain can be used for improving gestures performed by a human. For example, gestures for a precision work (e.g., watchmaker) can be improved if the brain is trained for controlling specific, precise gestures. As another example, the trainings of the athletes (e.g., sprinters), musicians and the like comprise repeating the same movements in order to improve their coordination and, more generally, their performances. In these examples, the trainings of the brains rely mainly of the repetition of the movements, repetition that enhances the subject's experience in doing the perfect gesture and performances. Acquiring this experience takes many years of practice and perseverance.

Within this context, there is still a need for an improved device for training the motor cortical areas of a human brain.

**SUMMARY OF THE INVENTION**

It is therefore provided a device for training motor cortical areas of a brain of a human subject. The device comprises:

- a display for providing the human subject with a set of visual stimuli;
- a speaker for providing the human subject with auditory stimuli;
- sensors for collecting responses of the human subject to the stimuli, the collecting comprising:

-- collecting with a first sensor data for measuring a cognitive response of the human subject, the cognitive responses comprising the reaction time and/or error estimation of the subject in response to the stimulus and the duration of both explicit and implicit mental simulation of displayed body movements of the set of visual stimuli;

-- measuring with a second sensor using electroencephalogram techniques, desynchronization of alpha waves of the brain, of low beta and Mu rhythm;

-- measuring with a third sensor kinematics of at least one part of the body of the subject;

- from the collected responses, estimating by a processing unit a motor adaptation of the subject in response to one or more of the provided stimuli.

The device may comprise one or more of the following:

- the estimating the motor adaptation of the subject comprises using a trained neural network to obtain an estimation of the motor adaptation of the subject, the neural network having been trained based on a dataset of collected responses of the subject to sets of visual and auditory stimuli, the neural network being configured for estimating a motor adaptation of the subject in response to one or more of the provided stimuli;
- the neural network having been further configured for estimating a dynamic of a training program of the motor cortical areas of the brain of the human subject along short and long-time frames;
- the dataset of collected responses and the sets of visual and auditory stimuli for the learning are obtained in a similar way as for the training;
- the collecting responses of the subject for the training is performed on the basis of short-time frames, each short-time frame representing a subject training session of up to 30 minutes; long-time frames, each long-time frame being computed by consolidating the data of the short time frames on a weekly and/or monthly basis;
- the visual and/or auditory stimuli are produced using computer graphics tools and methods for activating a perceptual property of the brain, the perceptual property being selected among a motor resonance, an affordance, a visual inference allowing mental simulation of action;
- the produced visual stimuli comprise images and/or videos with an impoverished appearance in structure and/or contour and/or color and/or speed and/or with an altered observer's point of view altered;
- measuring the reaction time comprises measuring a time lag between the actual end of a movement of the subject in response to the stimulus and an end of the movement estimated by the subject; and wherein measuring an error estimation comprises measuring an error in movement duration estimation of the displayed movement, and/or measuring an error in the subject's response to a visual stimulus when it must be associated with a sound stimulus and vice versa, an error occurring when an unexpected of response of the subject to the stimulus is performed;
- the measuring kinematics is performed with markerless techniques to extract kinematic biomarkers of the sensory-motor deficiency among which the maximum velocity, the time to peak velocity, the smoothness and the energy cost of the movement;
- if a lack of dexterity is detected in response to the measurement of a cognitive response of the subject, repeating the last stimulus; if a lack of dexterity is detected in response to the measurement of the kinematics of the subject, repeating the former stimulus; if a lack of dexterity is detected in response to the measurement of desynchronization by using the EEG techniques, repeating the first stimulus of the set of stimuli;
- the estimating motor adaptation of the subject in response to one or more of the provided stimuli comprises comparing a sensory-motor adaptation of the subject in response to one or more of the provided stimuli with limits of normal values of the sensory-motor adaptation of the subject in response to one or more similar stimuli, the limits of normal values being obtained from a large group of non-affected subjects;
- a memory communicatively coupled to the processing unit, the memory storing a computer program comprising instructions that when executed by the processing unit cause the device to estimate from the collected responses the motor adaptation of the subject in response to one or more of the provided stimuli;
- the first sensor comprises a sound recorder; the second sensor comprises EEG electrodes; the third sensor comprises two motion capture cameras; and wherein the computer program further comprises instructions, that when executed by the processing unit, cause the display to emit the set of visual stimuli, the speaker to emit auditory stimuli, and the sound record to record vocal responses of the subject;

It is further provided a computer program comprising the instructions stored on the memory.

It is further provided a computer readable medium storing the computer program..

## BRIEF DESCRIPTION OF THE DRAWINGS

Embodiments of the invention will now be described, by way of non-limiting example, and in reference to the accompanying drawings, where:

- FIG. 1 shows an example of the device;
- FIGs. 2 to 8a show examples of visual and auditory stimuli;
- FIGs. 9 to 13 show examples of data collections;
- FIG 14 shows an example of supervised training program;
- FIG. 15 shows a flowchart of an example of the method performed by the device of FIG. 1; and
- FIG. 16 shows an example of human subject learning curves.

**DETAILED DESCRIPTION OF THE INVENTION**

It is proposed a device for training motor cortical areas of a brain of a human subject. The device comprises a display for providing to the human subject with a set of visual stimuli; visual stimuli can be displayed to the subject. The device also comprises a speaker for providing the human subject with auditory stimuli. The device further comprises sensors for collecting responses of the human subject to the stimuli; the measurements are performed for each stimulus, and are collected. A first sensor for collecting data measuring a subject's cognitive response. A cognitive response comprises the reaction time and/or error estimation of the subject in response to the stimulus and the duration of both explicit and implicit mental simulation of displayed body movements of the set of visual stimuli. A sensor uses electroencephalogram (EEG) techniques and can measure the desynchronization of alpha waves of the brain, of low beta and Mu rhythm. A third sensor can measure kinematics of at least one part of the body of the subject. Then, the device estimates with a processing unit, from the collected responses, the motor adaptation of the subject in response to one or more of the provided stimuli.

Such device improves the training of motor cortical areas of the brain of a human subject.

The present invention notably relies on the widely accepted thesis and studies demonstrating that the human brain remains modifiable throughout life, and that structural reorganization via brain plasticity is optimizable. The present invention takes into account principles of motor cognition together with motor resonance. Motor cognition refers to that is embodied in action, and that the motor system participates in what is usually considered as mental processing. Motor resonance refers to the mechanism by which motor cortical areas are activate upon visual/auditory stimulations via the network of mirror neurons; for example, the observer's motor system is internally activated, specifically attuned to a perceived movement. The concept of embodied cognition is exploited in the present invention and therefore reduces the usual gap between training activities of the brain, and everyday motor skills. For examples, visual perception activates the motor cortex, which in turn predicts the sensory consequences of mentally simulated movement by the subject. The invention takes advantage of these concepts to optimize the stimulation of motor cortical areas of a human subject: indeed, the recording set up capture motor, cortical and cognitive activities of the subject, and the transformation of the subject's performance during the training programme is obtained from these three types of measurements.

In addition, the present device combines visual and auditory effects (also referred to as visual and audio stimuli). This allows to create a realistic sensory environment that encourages the transfer of learning to everyday training activities of the human subject. This contributes to improve the efficiency of the brain training. Furthermore, different sensory inputs facilitate the activation of motor areas which, under normal living conditions, systematically involve several sensory areas (proprioception, vision, tactile, vestibular...). Thus by providing measurements of subject's responses (e.g. cognitive, mocap, EEG) to the device, a virtual coach of the subject can be informed of the evolution of the subject's motor adaptation over hours, weeks, and even months; the difficulty of the proposed exercises can be adjusted, subject engagement may encouraged, e.g., an appropriate distribution of visual and auditory stimuli leads the subject to generate his own internal rewards, and optimize the subject's responses and motivation implicitly - i.e., unbeknownst to him and without recourse to his deliberative intention-. All these aspects of the training of the motor cortical areas of a brain are objectified thanks to the estimate motor performance of the subject by the measurements performed with the sensors; this notably allows an automation of the training program as a complement to additional care giver intervention.

Further advantages of the present device will be set forth in the detailed description.

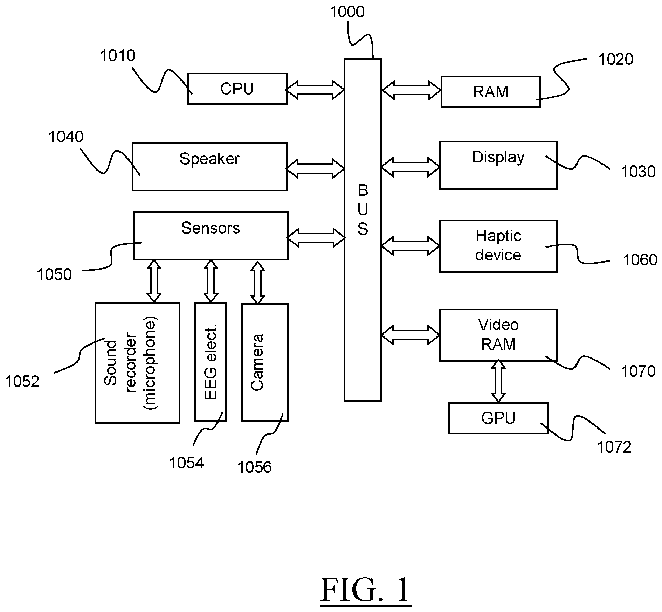

With reference to **FIG.** 1, it is shown an example of the device. The device comprises a processing unit or central processing unit (CPU) 1010 communicatively coupled (connected) to an internal communication BUS 1000. The device also comprises an access memory (e.g., a RAM) 1020 also communicatively coupled (connected) to the BUS. The device is further provided with a display 1030 to provide visual inputs to a subject that uses the device. In the example of **FIG. 1,** the device comprises a graphical processing unit (GPU) 1072 which is associated with a video random access memory 1070 connected to the BUS. Video RAM 1070 is also known in the art as frame buffer. The Video RAM and the GPU can prepare the data for the display 1030. The display 1030 is connected to the BUS, being understood that any type of connection may be contemplated. The device further comprises an element for emitting sound to the subjects; a speaker 1040 is provided with the device. Any type of connection between the speaking part and the other elements of the device is provided via a standard speech recognition system. The device also comprises one or more sensors 1050.

In examples, each sensor 1052, 1054, 1056 may be adapted to perform a particular type of measurements. The sensor 1052 may be a sound recorder used for measuring the cognitive response of the subject, e.g., the sound recorded are processed by a computer program able to identify the vocal response of the subject and then his/her reaction time and/or error estimation in response to the displayed stimulus. In the same wat, the duration of both explicit and implicit mental simulation of body movements is estimated via the vocal recognition sensor. The computer program can be stored on the memory 120 and executed by the CPU 1010 of the device. The sensor 1056 may be two cameras that are able to capture body movements of the subject in response to the stimulus, but without the need to add markers on the body of the subjects. Thus, the two cameras allow to reconstruct the hand and upper limb movement in 3D following the markerless method.

The sensor 1054 may comprise EEG (electroencephalogram) electrodes that measures desynchronization of alpha waves of the brain, of low beta and Mu rhythm. EEG provides a reliable reflection of the brain's electrical state. It is to be understood that the sensor 1056 may comprise an EEG machine that records the electrical activity of the brain and can contain electrodes that can detect brain activity when placed on a subject's body (e.g., the subject's scalp): the electrodes measure the brain wave patterns forming the data and sent them to an the EEG machine that receives the signals sent by the electrodes, may perform treatments on the signals, may record the measurements, and the EEG machine sends the data to the device. It is to be understood that any configuration/type of the EEG sensor 1054 can be contemplated.

In examples, the sensor 1056 may be one sensor, e.g., a camera. In these examples, the camera is used for measuring the kinematics of at least one part of the body of the subject in response to the stimulus. The images obtained by the camera may be processed by a computer program that measures the kinematics of the body of the subject from the images captured by the camera. This computer program can be stored on the memory 120 and executed by the CPU 1010 of the device.

In examples, the memory of the device of the present invention may store a computer program comprising instructions that when executed by the processing unit cause the device to estimate, from the collected responses, the subject's motor performance in response to one or more of the provided stimuli. The device thus collects the measurements that are stored and used by the computer for obtaining the adaptation of subject's motor response. Further examples of this computation will be discussed hereinafter; the adaptation of the subject's motor response reflects the transformation of the subject's motor response.

In examples, the device may further comprise a computer program comprising instructions that when executed by the processing unit cause the display to emit the set of visual stimuli and the speaker to emit auditory stimuli. In other word, the emission of the stimuli by the device are commanded by a computer program.

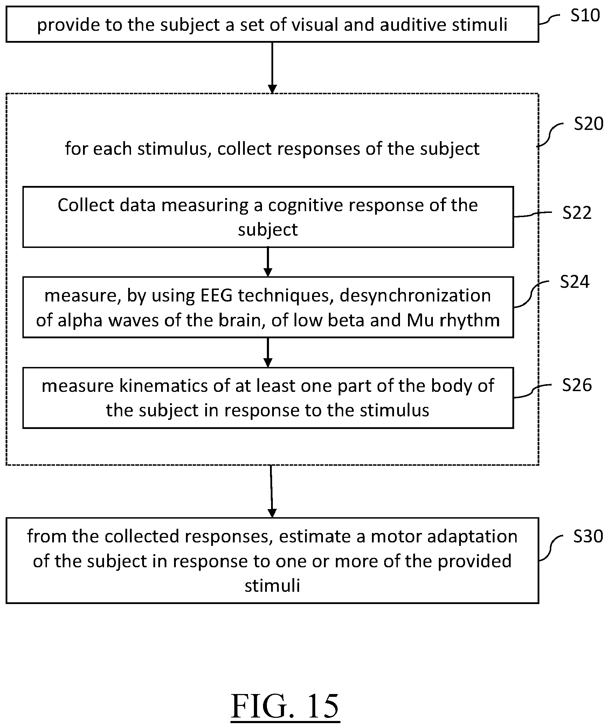

In examples, the device may further comprise a computer program comprising instructions that when executed by the processing unit cause the device to perform a method for training motor cortical areas of a brain of a human subject as illustrated on **FIG. 15.** The method comprises providing (S10) to the subject a set of visual and auditory stimuli. The method comprises, for each stimulus, collecting (S20) responses of the subject by -- measuring (S22) a cognitive response of the subject, the cognitive responses comprising the reaction time and/or error estimation of the subject in response to the stimulus and the duration of both explicit and implicit mental simulation of body movements; -- measuring (S24), by using electroencephalogram techniques, desynchronization of alpha waves of the brain, of low beta and Mu rhythm; -- measuring kinematics (S26) of at least one part of the body of the subject in response to the stimulus. And the method also comprises, from the collected responses, estimating (S30) the motor adaptation of the subject along a training program, e.g., in response to one or more of the provided stimuli. In other words, the computer program controls de devices and the operations performed by the elements of the device.

The steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or predefined.

In example, the device may also comprise a mass memory device, such as hard drive. Mass memory devices suitable for tangibly embodying computer program instructions and data include all forms of nonvolatile memory, including by way of example semiconductor memory devices, such as EPROM, EEPROM, and flash memory devices, magnetic disks such as internal hard disks and removable disks, magneto-optical disks, and disks. Any of the foregoing may be supplemented by, or incorporated in, specially designed ASICs (application-specific integrated circuits). A network adapter may manage accesses to a network. The client computer may also include a haptic device 1060 such as cursor control device, a keyboard or the like. A cursor control device is used in the client computer to permit the subject to selectively position a cursor at any desired location on display 1030. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively, or additionally, the client computer system may comprise a sensitive pad, and/or a sensitive screen.

The computer program(s) may comprise instructions executable by a computer, the instructions causing the device to perform the above method or causing the display to emit the set of visual stimuli and the speaker to emit auditory stimuli and/or causing the device to estimate, from the collected responses, the motor adaptation of the subject in response to one or more of the provided stimuli when the instructions are executed by the processing unit.

The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method.

Back to **FIG. 1,** further examples and explanations of the devices are now discussed.

The device comprises a display for providing the human subject with a set of visual stimuli, and a speaker for providing the human subject with auditory stimuli. Thus, a set of visual and auditory stimuli is provided to the human subject. By set of stimuli is meant a sequence of visual and auditory stimuli specifically designed to activate the motor cortex of the brain.

In examples, the visual stimuli are produced using computer graphics tools and the auditory stimuli are produced using computer audio tools. The visual and auditory stimuli are produced with methods for activating perceptual property of the brain. These methods for activating a perceptual property of the brain may comprise methods that exploit a motor resonance and/or an affordance and/or a visual inference allowing mental simulation of an action; preferably these three perceptual properties may be exploited, e.g., in combination. In an example, the visual stimuli are produced using a video viewer of daily human action or of human actions reconstruct via computer graphics tools, and the auditory stimuli are produced using computer audio tools or via recorded natural sound of action.

Motor resonance is exploited via mirror neurons, and it relies on an automatic imitation without execution of an observed or listened-to act. Mirror neurons are a kind of neuron that both when the subject acts and when the subject observes the same action performed by another. The subject watches a video or an image showing a person performing an action, and the subject mentally performs but not physically. Mirror neurons essentially react to a visual stimulus that represents an interaction between a biological means of action (hand or mouth) and an object. They therefore behave as neural substrates for cognitive processes like the recognition of an action rather than a simple movement. A large body of experimental evidences demonstrated that visual perception of human actions elicits activation within a brain network closely related to that one that participates in motor execution; the document "The mirror-neuron system, Annual Review of Neuroscience, Vol. 27:169-192 (Volume publication date 21 July 2004)" defines mirror neurons.

Affordance that characterizes a tight coupling between perception and action, describes what a subject is capable of doing with an object before we actually do it. Thus, the visual perception of an object evokes its handling and identifying its function. Similarly, the auditory perception of a known sound evokes to the user an object/event and its function. Affordance accurately reflects the ability of humans, and animals in general, to guide their behavior by perceiving what the environment offers in terms of potential actions. The document « Gibson, J. J. (1979). An ecological approach to perception. Boston (MA): Houghton Mifflin" discusses and give definition of affordance.

Visual inference refers to the sudden interruption of visual inputs during the visual pursuit of a movement is compensated for by the subject's mental simulation of the action occluded. Theories of embodied cognition emphasize that incomplete visual perception of action is compensated for by cortical representations of bodily experiences. Inference refers here to the capacity to reconstruct the missing trajectory of, for example, a hand movement and extrapolate the final position of the hand that suddenly vanishes behind a wall. Studies showing maintenance of cortical activity after objects in motion disappear indicates that internal model of action are recalled to reconstruct the missing part of the trajectory, as discussed in Pozzo et al. 2006; Kinematic features of movement tunes perception and action coupling, in Behavioural Brain Research, Volume 169, Issue 1, 25 April 2006, Pages 75-82.

Hence, the visual and auditory stimuli activate at least one of these three perceptual properties of the brain; preferably these three perceptual properties of the brain are activated by a stimulus.

Examples of stimuli are now discussed in the following paragraphs. It is to be understood that the stimuli for activating the motor resonance and/or the brain affordance and/or the visual inference allowing action simulation are not limited to the following examples. The visual or auditory environments created by the stimuli are designed to potentiate the implicit mental evocation of the movement by forcing the subject to extrapolate the missing part of the gesture, to find the action corresponding to the perceived sound, to mentally simulate grasping the object... and thus increase activations of motor cortical areas.

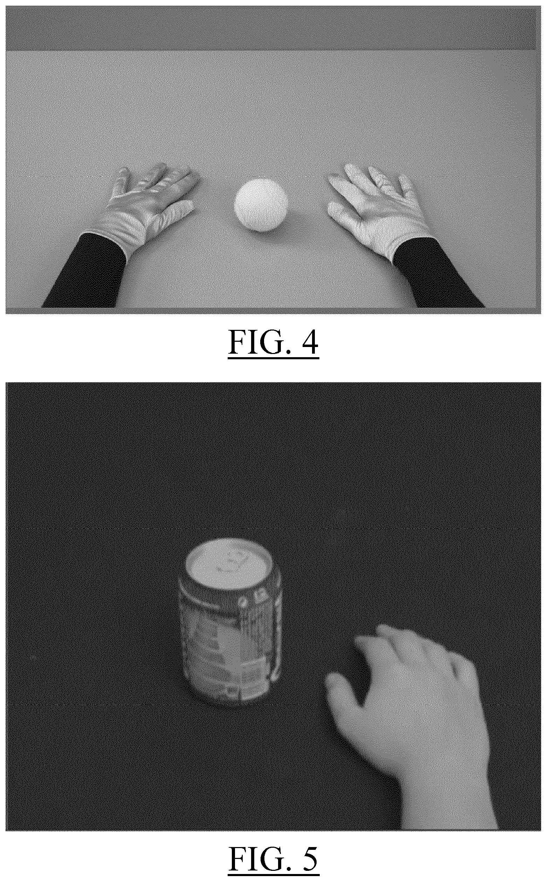

The situation of **FIG.** 4 and 5 aims at stimulating the motor resonance of the subject's brain and are example of video of grasping action displayed on the screen of the computer to activate the mirror neurons system.

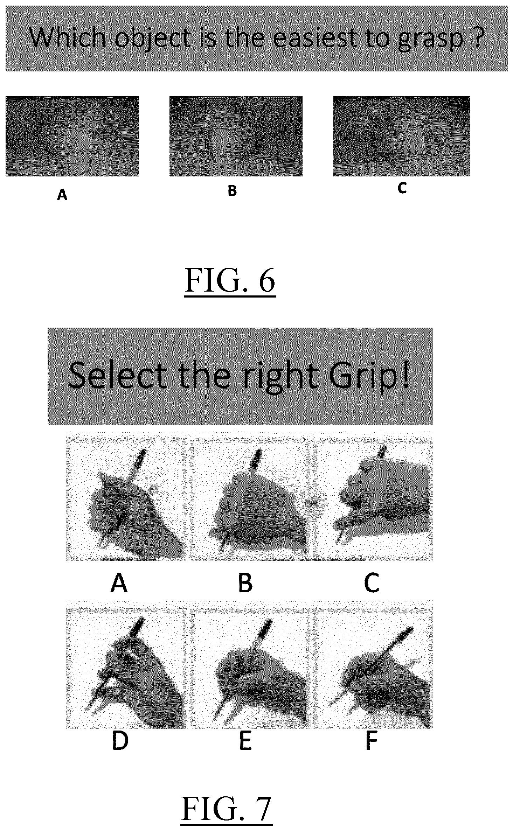

The situation of **FIG. 6** aims at stimulating the affordance property of the subject's brain. The image of a graspable object (here a tea pot) is displayed in three orientations and the subject is asked to estimate which one among the three tea pots is easiest to grasp. The user can provide his/her answer by selecting on the three images with the haptic device 1060.

Similarly, to **FIG. 6, FIG. 7** aims at stimulating the brain affordance of the subject. Six grasping postures of a same gesture (grabbing a pen) are presented with natural or artificial grasping styles and the subject is asked to estimate which is more natural. For instance, the subject uses the vocal recognition device 1052 and/or the haptic device 1060 for selecting the response of their choice during the cognitive collection, by a vocal response to cognitive tasks (a phonemes like "A" "B" or "C" ... depending on the number of possible responses, or "Go"/ "Stop" or Yes/No), or by clicking with a cursor on the selected response.

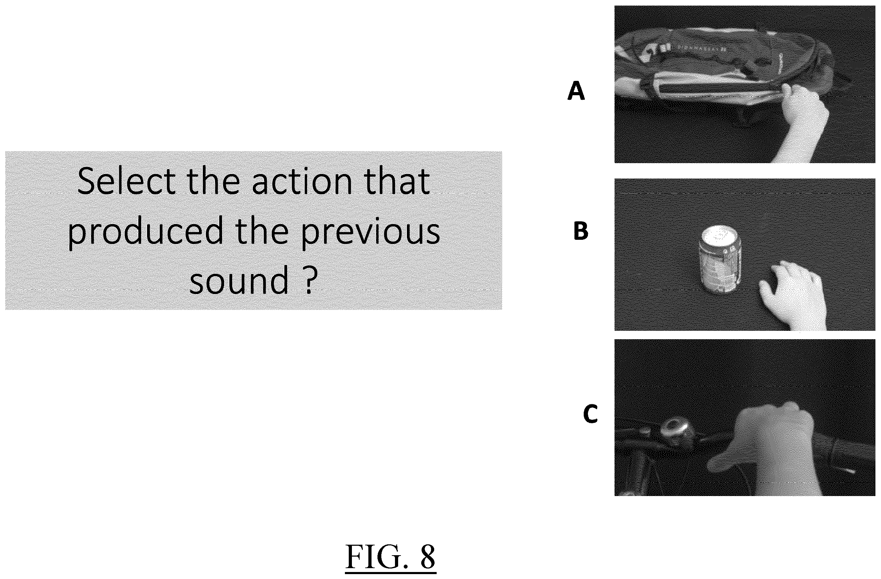

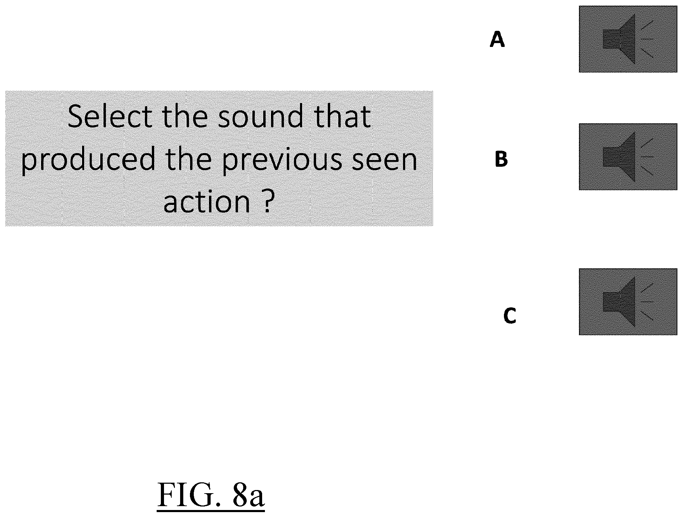

**FIGs. 8** and **8a** illustrate the object from which an auditory stimulus for activating the motor resonance (or the reverse, that is a visual stimulus to infer the corresponding sound, **FIG.8a).** A sound is emitted by the device while three images each representing an object are displayed, and the subject has to select the object representing the action associated with the sound. Hence, the subject has to find the right corresponding object or sound, on the basis respectively of the sound he has heard or the object he has seen.

As another example, a motion displayed to the subject is interrupted according to the principle of visuo-motor inference and when trajectories are temporarily masked by a visual obstacle. The start of a movement is displayed after a countdown, then masked at a fixed and variable instant, and the subject must estimate when the masked movement will stop by a "stop" vocal response.

In examples, the produced visual stimuli may comprise images and/or videos with an impoverished appearance (e.g., a stick diagram instead of a full video of action). This maintains the subject's attention and active engagement in a process of motor simulation preceding his response as visual or auditory embellishments do not disturb it; that is, the brain does not need to interpret embellishments. In example, the impoverished appearance may be obtained by modifying structure and/or contour and/or color and/or speed and/or with an altered observer's point of view altered.

The measurements performed by the device on the subject are now discussed. For each stimulus provided to the subject, one or more responses of the subject are collected, that is, measured and stored for a subsequent analysis and/or use. Three types of measurements can be performed for each stimulus. It is to be understood that each stimulus does not necessarily have to trigger the measure of each of these three types of measurements. In addition, it is to be understood that no measure may be obtained for a given stimulus depending on the circumstances (e.g., physical and /or mental state of the subject). In any case, at least one of these three types of measurements is performed for each stimulus. Preferably, the sensors performing the measurements may be in state that enables them to make a measurement each time a stimulus or a question is provided to the subject.

The first measurement is collecting by a first sensor data for measuring a cognitive response of the subject (S22). Examples of the first sensor have been discussed in reference to **FIG. 1.** The cognitive response of a subject to a stimulus may be objectified by measuring a) the reaction time to a question asked to the subject as in the example of **FIG. 6;** and/or b) the error estimation in duration of the subject in response to the motion inference task; and/or the error in the selection of an answer among two or three proposed options. In addition, the cognitive response is also objectified by measuring the duration of both explicit and implicit mental simulation of body movements.

The measurement of the cognitive response of the subject with the reaction time and/or error estimation in duration, or error in the selection of an option of the subject in response to the stimulus provides a good accuracy. This accuracy may be improved by additionally measuring the duration of both explicit and implicit mental simulation of body movements. Mental simulation is the process of self-projection into alternate temporal, spatial, social, or hypothetical realities is a distinctively human capacity. An explicit mental simulation means a mental simulation based on what the subject is explicitly instructed to imagine.

An implicit mental simulation means a mental simulation based on what the subject is implicitly instructed to imagine. Explicit simulation involves mental imagery while implicit simulation requires neither 'consciously experienced analogue reasoning nor explicit episodic recall'. For instance, when the subject has to estimate whether a displayed object (e.g., the handle of a teapot oriented in 3 different directions as shown on **FIG. 6)** is easy (in this case the handle orientation is aligned with the observer's hand posture), or difficult (in this case a large angle separates the handle orientation from the observer's hand), the task (to give the answer) involves an implicit and automatic mental simulation of observer's hand rotation toward the different handgrip orientations. To estimate the difficulty of the grasp, or to answer a question (e.g., "right or left hand?"), the observer must mentally simulate the grasping gesture, a cognitive task classically considered as reflecting the good functionality of hand motor cortical areas. The measurement consists in calculating the response time that is the duration separating the appearance of the image on the screen from the vocal response of the subject saying A (left hand) or B (right hand) or given by a pressure on a key of the keyboard in case of non-vocal recognition. The response time calculated in millisecond is normalized in reference to the performance of a healthy subject on the same task provides the status of the mental simulation of the subject: 0 for errors in duration = $\pm$ 50%, 1 for errors = $\pm$ 30%, to 2 points for errors = ± 10%). The cognitive performance is also estimated on a scale 0/1 scale: invalid/0 or valid/1 response, and 0.5 if a good answer given beyond a maximum duration.

Other examples of mental simulation of action are now discussed for the sake of illustration:

- Natural speed estimation: the video of an arm reaching is displayed at different speeds (normal, fast or slow): the subject has to select the most natural (or the slowest or the fastest) speed by vocal ("A" or "B" or C) or pressing a key on the keyboard. The performance is estimated on a 0/1 scale: invalid or valid response, and 0.5 if a good answer given beyond a maximum duration.
- Audiovisual coupling: the video of an object manipulation displayed on the screen is followed by an audio stimulus (or vice versa, that is an audio and then a photo of an object) corresponding or not to the sound (or to the video) produced by the action. The subject should indicate if the following sound (or object) agree with the audio/video stimulus by vocal (Yes or No). In this case it is the validity of the answer that is recorded on a 0/1 scale (invalid or valid answer) and 0.5 if a good answer given beyond a maximum duration.
- Guess a hidden object: the video of a grasping movement towards an invisible object and whose final phase of movement is hidden, is displayed on the screen. The subject then has to select, among 3 objects of different size/shape, the one that corresponds to the hidden object to be grasped by vocal A, B or C (if there are 3 possible objects to be selected). In this case it is the validity of the answer that is recorded on a 0/1 scale (invalid or valid answer) and 0.5 if a good answer given beyond a threshold maximum duration.
- Motion inference: the video of a grasping movement in which the final phase (or an intermediate phase) is hidden, is displayed on the screen. The subject who has to initiate and determine by vocal (Go&Stop) (or pressing a key of the keyboard) the instant he/she estimates to correspond to the contact of the hand with the invisible object to be grasped; or the instant of reappearance of the hand that has been transiently masked. The duration estimation error is calculated in msec and then normalized with reference to a nominal value calculated with a population of healthy subjects, on a 0/2 scale: 0 for duration errors of ± 50%, 1 for errors of ± 30%, to 2 for errors ± 10%.
- Impoverished video and Motion or object recognition: Display a PLD (or a full video) and indicate by vocal (Yes or No), or key press, if the following video (PLD) agrees with the previous PLD or video (the PLD can be displayed with different quantity of PLD to modulate the difficulty of the task). Scoring: validity of the answer that is recorded on a 0/1 scale (invalid or valid answer) and 0.5 if answer given beyond a maximum duration.

The reaction time evaluate the level of subject's attention. Baseline subject's Reaction time (RT) may be calculated at the beginning of each data collection for measuring a cognitive response of the human subject, e.g., at the beginning of each training session of the subject. In examples, RT is calculated by measuring the reaction time when the image of a cross on the screen (or a sound) appears at the end of a countdown clock displayed on the screen: the time separating the beginning of the display (end of the countdown) of the cross from the subject's response vocal (Stop!) or given by pressing a key on the keyboard is calculated. The RT also provides information on the adherence and commitment of the observer to follow the learning program. The RT is calculated in millisecond and then normalized in reference to the individual baseline recorded at the beginning (and also to the mean performance of healthy subjects on the same task) according to a scale ranging from 0 to 3: RT > 50%=0 ; 50%>RT> 30%=1; 30%>RT> 10%= 2; RT<10%= 3).

The second type of measurement comprises measuring the desynchronization of alpha waves of the brain, the desynchronization of low beta and the Mu rhythm (S24). Cortical activity is recorded via EEG during observation of video of action in order to monitor the activation of motor-related cortical resources. Action observation elicits a well-established pattern of EEG activity in central electrodes known as mu ($\mu$) (8-13 Hz) suppression, which is commonly used to index the activation of the mirror neuron system, as discussed for example in H. Hobston et al., The interpretation of mu suppression as an index of mirror neuron activity: past, present and future, in Royal Society Open Science, March 2017, Volume 4, Issue 3. Electroencephalogram (EEG) techniques are used and involve EEG sensors. EEG sensors have been discussed in reference to FIG. 1. The second type of measurements is performed as known in the art; for example, in the document S. Boni et al., Action Observation Therapy for Arm Recovery after Stroke: A Preliminary Investigation on a Novel Protocol with EEG Monitoring, in J. Clin. Med. 2023, 12(4), 1327.

The third type of measurement comprises measuring kinematics of at least one part of the body of the subject in response to the stimulus (S26). This is performed using a third sensors and examples have been discussed in reference to **FIG. 1.** Kinematics mostly describes the motion of human body in space, not the forces that cause that motion. The third sensor thus measures motions of body parts (located via specific anatomical landmarks) of the subject, e.g., the distance covered by the subject's wrist during hand movement to be performed during the Mocap phase of the training program.

In examples, the kinematics measurement is performed with markerless techniques for estimating the poses of (part of) the body of subject. In an example, the kinematics measurement is performed with markerless Deeplabcut™ technique that is an efficient method for 2D and 3D markerless pose estimation based on transfer learning with deep neural networks that achieves excellent results with minimal training data, as discussed for example in A. Mathis et al., DeepLabCut: markerless pose estimation of user-defined body parts with deep learning, in Nature Neuroscience volume 21, pages1281-1289 (2018). Markerless techniques allow extracting kinematic biomarkers of the sensory-motor deficiency. In examples, the maximum velocity of subject's hand movement is measured. Alternatively, or additionally, the time to peak velocity is measured. Alternatively, or additionally, the smoothness of the movement performed by the subject is measured. Alternatively, or additionally, the energy cost of the movement is measured. These measurements are performed as known in the art, as discussed for example in B. Berret et al., Evidence for Composite Cost Functions in Arm Movement Planning: An Inverse Optimal Control Approach, October 13, 2011.

In examples, a score may be computed for the one or more collected responses to stimulus or for the responses collected to stimuli. The score of a measure (or a group of measurements) is a summary indicator describing the observed events. Hence each of the cognitive response(s), EEG indicator(s) and kinematics of the subject is associated with a score. The calculation methods of the score(s) may be any of those that are known in the art. In an example, for each cognitive and motor task, the subjects' response is normalized and converted to a point scale. A daily score of the attention level and mental simulation may be recorded in a database. The performances of the subject may be then reported at the end of the bloc in the form of a visual feedback on the screen which informs the subject about his progress during the recovery process. The progress may be estimated by calculating the Subject Relative Change (referred to as PRS). The PRS value for a variable X (among normalized Cognitive and Mocap variables) is calculated as follows:

- if a higher value for X is an improvement of the subject's performance, the formula (1) is used:

$$PRSx = \frac{100\,(Xt2 - Xt1)}{\max(100 - Xt1, 0.001)} \quad (1)$$

- if a higher value for X is a degradation of the subject's performance, the formula (2) is used:

$$PRSx = \frac{100\,(Xt1 - Xt2)}{\max(Xt1, 0.001)} \quad (2)$$

where for formula (1) and (2) Xt1 and Xt2 are the values of the collected variable at time points T1 and T2, respectively. The max (..., 0.001) function is included in order to cope with edge cases, where the value at the start of the rehab is already optimal (i.e., no further improvement is possible), as otherwise PRS would be undefined due to a division by zero. The collection of the human subject responses to the stimuli is now discussed. Collecting a response to a stimulus means that at least one sensor has obtained at least one measure and that the at least one measure has been sent to a memory of the device for storage.

In examples, the device performs the collection on the basis short-time frames. Short-time frames refers to a training session of the subject that last up to 30 minutes. The purpose of the short training is to measure the responses to the stimuli as there are provided to the subject. Especially, short-time frames consider implicit learning (subject's adaptation without deliberate intentions form them) as more modifiable and effective than explicit strategies. The device further performs the collection of the measurements on the basis long-time frames. A long-time frame is computed by consolidating the measurements of the short-time frames on a weekly and/or monthly basis. Long-time frames thus provide consolidated information regarding the improvement of the subject's brain as a result of past training (e.g., short-time frames). Especially, long-time frames provide information regarding the explicit learning (subject's adaptation with deliberate intentions from them that can be referred to as metacognitive through subject's deliberation). Consolidating the measurements means that a new value representing a set of values of the short-time frames is computed, e.g., the new value of the long-time frame is an average on the values of the short-time frame for a period of time considered -one or more days, one or more weeks, one or more months, on or more years).

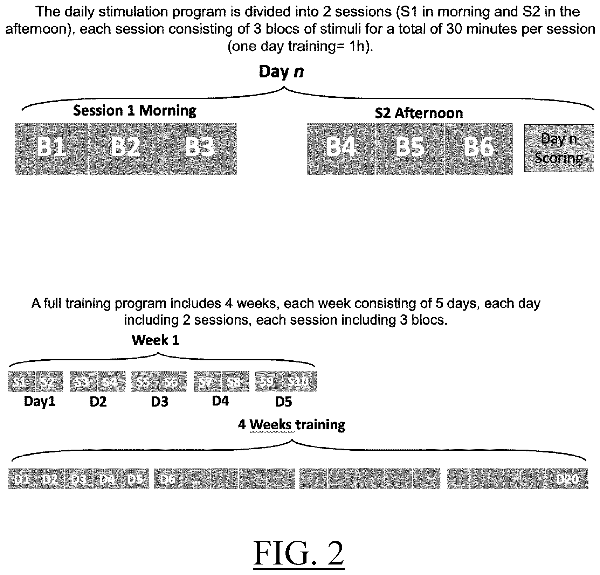

Referring now to **FIG. 2,** examples of providing the human subject with sets of visual stimuli and providing the human subject with auditory stimuli are discussed. Here the providing is performed according to short-time and long-time frames, being understood that the collecting will be performed at the same time.

**FIG. 2** shows an example of a training program. A bloc of stimuli (B1, B2,..., B6) represents the basic unit of the rehabilitation program, each bloc consisting of for instance of ten visual or auditory stimuli, within which 3 cognitive data collections and 2 Mocap (at t0 and tf) are interspersed. These quantities can be modulated with respect to the subject's status in term of attentional and motor performance via the virtual coach and the supervised training method. The daily stimulation program is divided into 2 sessions (S1 in morning and S2 in the afternoon), each session consisting of 3 blocs of stimuli for a total of 30 stimuli and 30 minutes per session (one day training thus represents 1h of training) for example. A full training program includes 4 weeks, each week consisting of 5 days, each day including 2 sessions, each session including 3 blocs and each bloc could including 10 stimuli. For example, a full training program can include for instance include up to 1200 stim (audio or video), up to 360 Cog evaluations and up to 240 Mocap evaluations. EEG recording is performed along a full bloc duration.

A video provides visual and auditory stimuli. Interestingly, each subsequent session starts with the last video of the former session; this allows to measure an improvement or a deterioration of the subject in response to same stimuli (a video). It is to be understood that the first and second training programs may be merged; each session comprising four videos and two questions.

Still in reference to **FIG. 2,** each session (B1+B2+B3 in the morning and B4+B5+B6 in the afternoon) is a short-time frame of the training and that the collection of the measure is made on the basis of that short-time frame. Each day (D1, D2...D5) is a long-time frame of the training and the measurements of the sessions of each day are consolidated, thus forming a daily long-time frame of consolidated collected measurements. Similarly, each week (W1... W4) a long-time frame of the training and the data of the sessions of each day are consolidated for each week, thus forming a weekly long-time frame of consolidated collected measurements of each session or of each day.

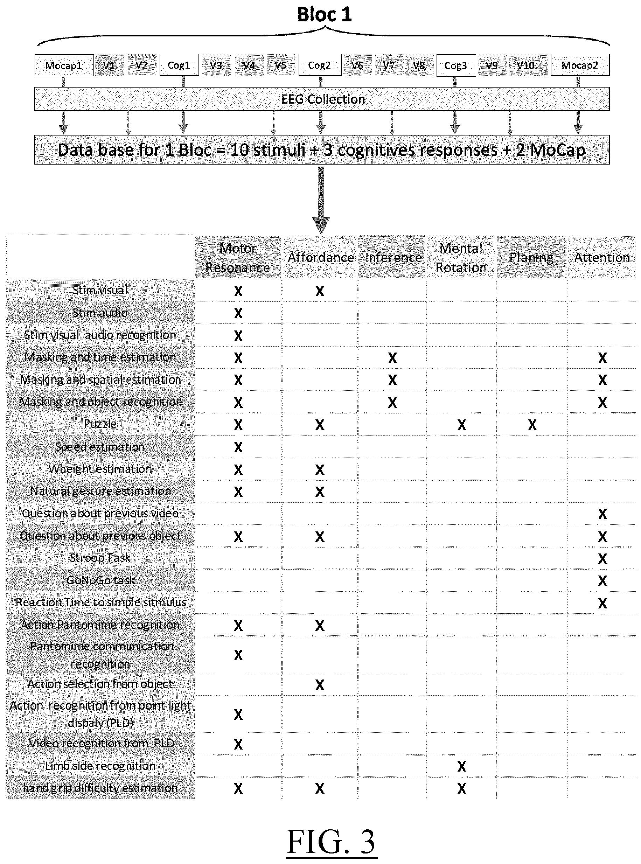

Referring now to **FIG. 3,** a table resuming examples of stimuli (leftmost column) triggering a cognitive response are provided, thus collected and then evaluated as discussed hereinabove. The cognitive response may involve the three perceptual properties of the brain already discussed. In addition, the stimuli may be used for measuring additional cognitive responses of the brain, including at least the mental rotation of body parts, the planification capacities of the brain (e.g., the capacity of the brain to correctly ordered action sub-phases, like first grasp the glass with one hand then grasp the bottle with the other hand and finally pour the water in the glass), and the attention of the subject which is the capacity to concentrate. At the time of the collecting, measurements may be stored so that, for a given type of stimulus, the values of the measure are stored together with the type of cognitive response triggered by the stimulus. Alternatively, or additionally, a score of each measure may be computed and stored with the associated measure, as discussed hereinabove. Alternatively, or additionally, a score may be computed and stored for each kind of cognitive response (the columns in FIG. 3), e.g., for each session and/or each day and/or each month and/or each year. On top of FIG. 3 is represented the storing of a collection of cognitive responses, the collection thus forming a block (block 1). While stimuli are provided to the user (e.g., videos are displayed), two motion captures (Mocap1, Mocap2) are performed at the beginning and the end of the block 1 collection, cognitive responses (Cog 1,...,Cog3) are collected, and ten EEG responses are measured (one for each stimuli). For block 1, are stored the ten EEG stimuli, the three cognitive responses and the two Mocaps.

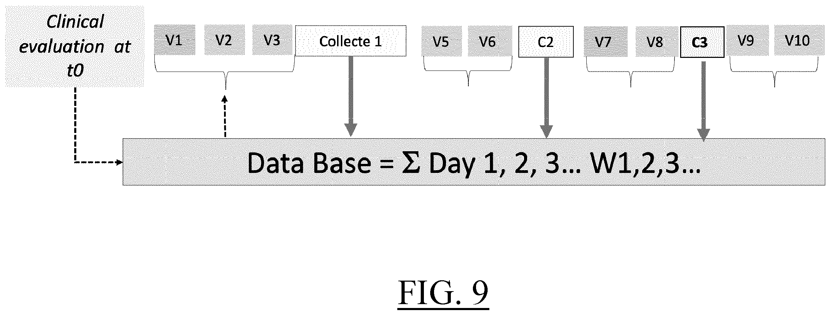

Referring now to **FIG. 9,** examples of collecting the responses are discussed.

The collection is shown for one bloc of one session training day, e.g., one of the days D1...D5 of a week W1...W4. Here, the measurements are collected three times (C1...C3) at the end of each two or three videos displayed. Said otherwise, collection and storing on the device of the measurements is carried out for each short-time frame. The short-time frame measurements are then consolidated in order to compute long-time frame measurements, e.g., the measurements are stored in a database -more generally any storage element allowing to retrieve structure, semi-structure or raw data or information-. When two or more measurements for a same type of stimulus have been collected, an average may be computed, replacing (or alternatively being stored with) the set of measurements obtained: by this way, measurements of sessions that given day are thus consolidated. Similarly, a table may be filed out with a new measure representing the measurements of the sessions of the training days of one week. Measurements of sessions of a week are thus consolidated. Similarly, a table may be filed out with a new measure representing the measurements of the sessions of the training days of the training weeks of a month. Measurements of sessions of a training month are thus consolidated, as illustrated in the example of **FIG. 2** that sum up the number of data collection inside a bloc, a day, a week and a full training session of four weeks.

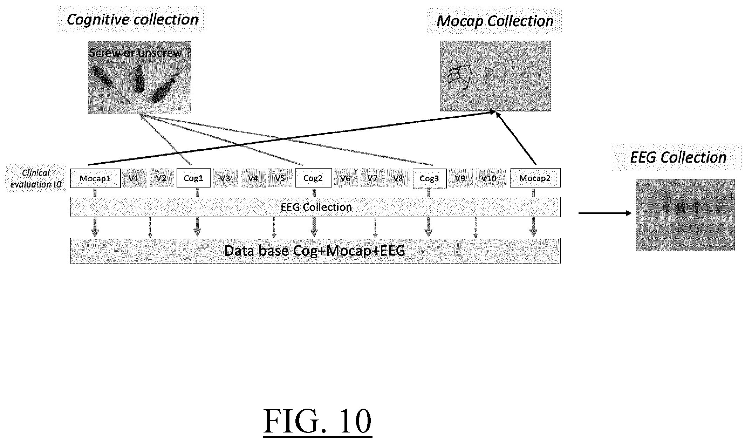

**FIGs. 10** to **13** show details of **FIG. 9.** On **FIG. 10** are represented the three types of measurements that are collected, namely cognitive responses of the subject, EEG measurements, and kinematics of at least one part of the body of the subject. The measurements of the cognitive responses to the stimuli are collected alternatively along the successive videos displayed in each bloc. The measurements of the motor responses to an imitation task are collected at the beginning and the end of each bloc. The recording of the EEG response may start from the beginning of the training and measurement of cortical activity is analyzed during each action video displayed. In the example of **FIG. 3,** a score may be computed for each training session and/or a score may be computed for the set of training session of a training day; training sessions scores and/or daily training scores are computed. A weekly score of the subject may be computed from the daily scores. Similarly, a monthly score may be computed from the weekly scores. Possibly, a yearly score may be computed from the monthly scores.

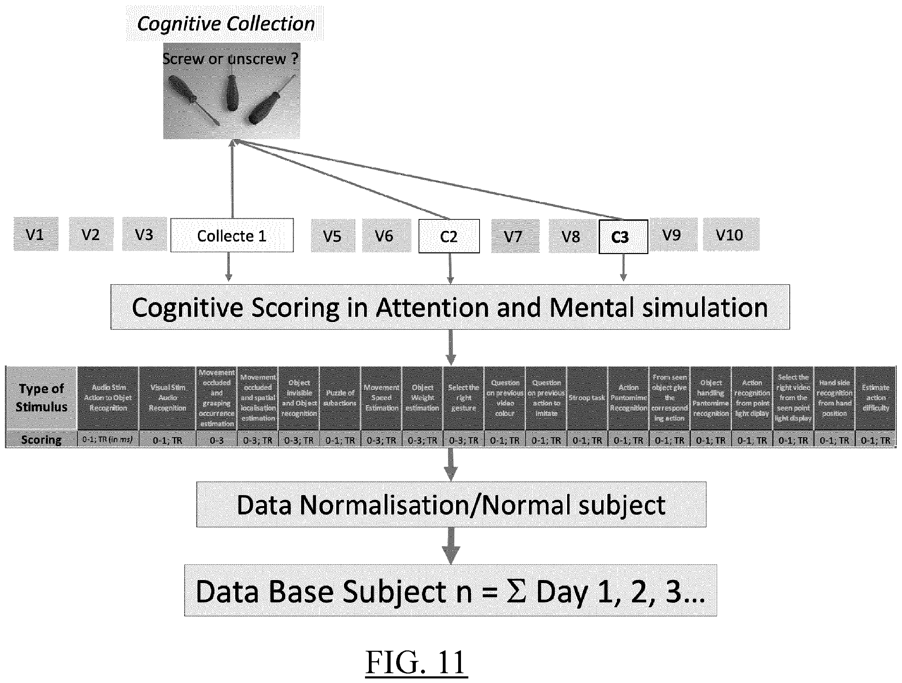

In reference to the examples of **FIGs. 11** to **13,** score computing for each type of measure is now discussed. Scores are computed for each of the three types of measurements, independently, even if the measurements may be collected during the same periods (C1...C3).

**FIG. 11** shows an example of measurements performed for cognitive responses of each short-time frame of a daily training. For each type of stimulus, such as those represented on the left column **FIG. 3,** a measure has been collected. For each of these measurements, a score is computed. A daily score is computed from the training session scores, and an average cognitive score of a subject can be computed with the formula:

$$Cog\overline{P_1} = \text{Day } 1+2+3+..n/n$$

with n that is the number of days for which a score has been computed. This score can be normalized (noted $P_1$) with the formula:

$$P_1 = Cog\overline{P_1}/Cog\overline{N}$$

CogN represents a cognitive score of the cognitive response of a standard subject. It is to be understood that the standard subject for normalizing the cognitive score may depends on the subject and/or on the training.

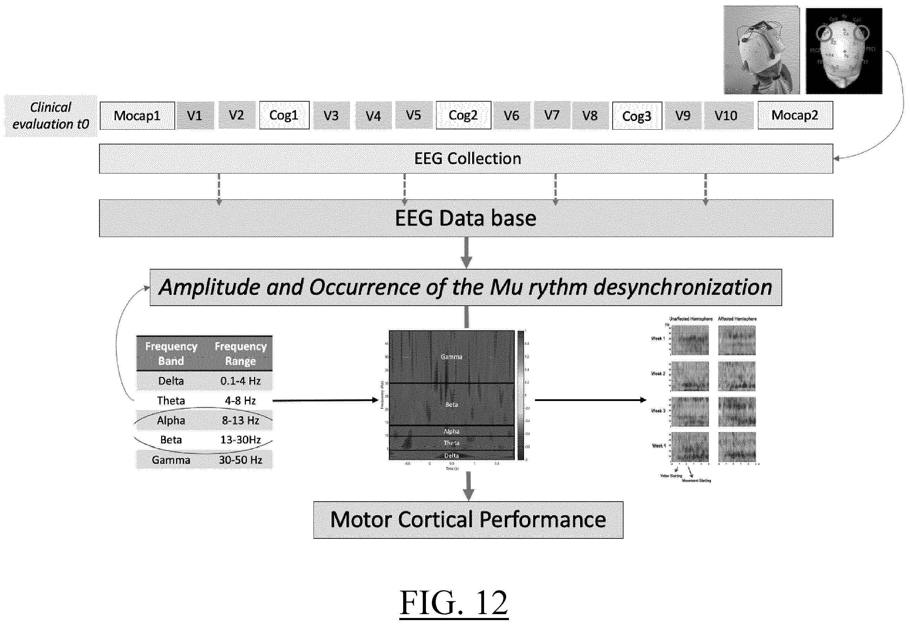

**FIG. 12** shows an example of EEG measurements obtained for a visual stimulus. The desynchronization of alpha waves of the brain, low beta and Mu rhythm are collected during each video displayed (e.g., when the observer is immobile), stored, and consolidated. More precisely, in this example, for each of these frequency band of the subject's brain, amplitude and occurrence of desynchronization are stored at least on a session basis. The consolidation is performed on daily basis so that an average of the measurements of each of the frequencies are computed and stored. A daily score is computed from the training session scores, and an average EEG score (noted eNeuro) of the subject can be computed with the formula:

$$\overline{eNeuro} = \text{Day } 1+2+3+..n/n$$

with n that is the number of days for which a score has been computed. This score can be normalized (noted $P_1$) with the formula:

$$P_1 = eNeuro\overline{P_1}/eNeuro\overline{N}$$

where eNeuroN represents an EEG score of the EEG response of a standard subject. It is to be understood that the standard subject for normalizing the EEG score may depends on the subject and/or on the training.

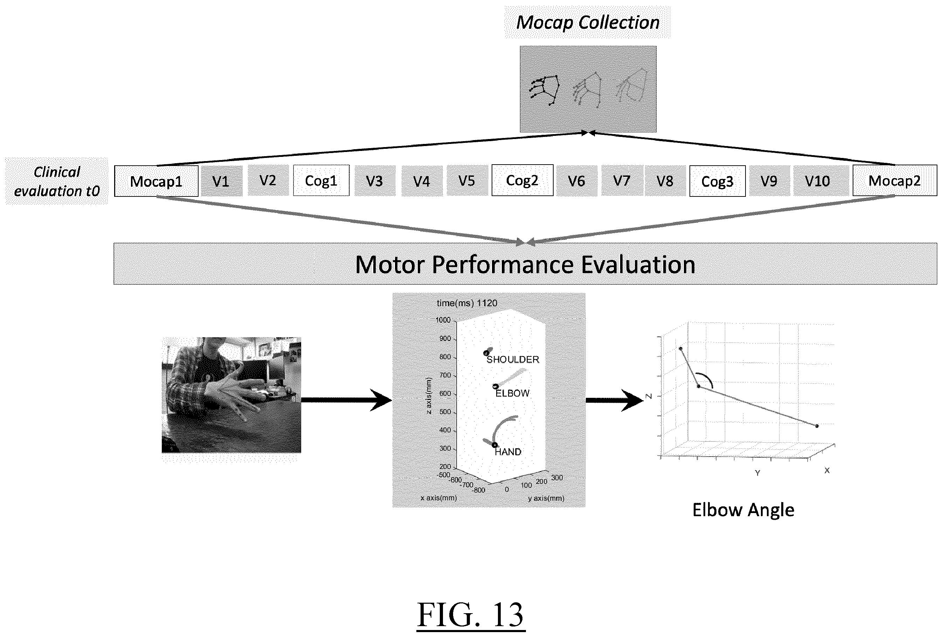

**FIG. 13** shows measurements of kinematics of the subject moving his arm when performing one of the gestures, for example as in the grasping action described in **FIG. 5.** In this example, the movement in the 3D space of the hand, elbow and shoulder of the subject are measured, an elbow angle as well. These measurements are collected and stored for each short-time frame (the training session). A consolidation may be performed on daily basis so that an average of the kinematics measured are computed and stored. A daily score is computed from the measurements (e.g., from the consolidated measurements); for example, a normalized kinetics score is computed using kinetics responses of a standard subject. It is to be understood that the standard subject for normalizing the kinetics scores may depends on the subject and/or on the training.

Back to the device depicted on **FIG. 1,** the collected responses are then processed (e.g., by a processing unit communicatively coupled to the memory storing the collected data) for estimating the motor adaptation, thereby obtaining a motor, cognitive and cortical performances of the subject in response to one or more of the provided stimuli. The motor adaptation estimation, and thus these performances are computed on the basis of the measurements of the responses. A global score is obtained from the measurements (e.g., for a training session), and this score is compared with at another one that had been previously computed for the same subject and/or from a theoretical score expected for the subject.

In examples, the motor recovery of the subject is estimated by comparing the subject's score in response to one or more of the provided stimuli with limits of normal values (statistically established in normal subjects) of the subject's response to one or more similar stimuli. The said normal subjects typically form a group of so-called non-affected subjects.

In examples, the pattern of motor adaptation of the subject along a training program may be obtained using a trained neural network, e.g., using machine, deep reinforcement learning. The training of the neural network is performed based on a dataset of responses of subjects to sets of visual and auditory stimuli. The training dataset is built with data similar to the one collected as explained hereinabove. For example, the dataset is built by collecting data on several subjects; the dataset may be obtained in a similar way as for the online inference. The trained neural network is configured for estimating a motor adaptation of the subject in response to the one or more of the provided stimuli. It is to be understood that the collected responses (used for the online inferences) car be used for training the neural network (for instance offline).

Hence, the collected responses of human subjects to stimuli feed a predictive model whose accuracy is proportional to the quantity of measurements made on the subject's responses.

This is based on the construction of a set of events taking place over short-time temporality {responses to stimuli in minutes) and long temporalities (weekly and monthly performances, in hours, days and weeks). Collection of the measurements will be discussed hereinbelow.

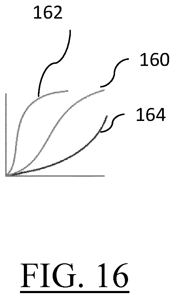

The modelling of the subject's sensory-motor adaptation is based firstly on EEG data, which can be collected in sufficient quantity at the end of the first bloc of a session. The modelling of the subject's daily response with EEG data is then enriched with cognitive and motor data collected over 5 days of training. A profile of subject is then approximate among 3 different pattern of sensory motor adaptation. This is illustrated on FIG. 16 that is discussed hereinbelow.

The trained neural network (or trained model) may be further trained for adapting the training (that is, the stimuli that are provided to the subject) to the response that are collected. Thus, for the short-time frames, data from subject's responses are used to adjust, during online inference, the difficulty of exercises to the singularities of subject's responses. The neural network predicts subjects' behavior and personalizes the training.

In examples, the personalization of the training may depend on a categorization of the subject: the subject is labeled depending on their response to the stimuli, the neural network assesses their progression and add a new label to the subject in response to the progression. The progression may be assessed by computing a score to the collected responses (e.g., for each training session) and comparing the latest computed score with the former one, or with the latest scores. Each label is associated with a training program and the difficulties of the training program depend on the label.

In examples, the neural network may adapt the training program online. This means that the neural network provides the subject with stimuli that are selected according to the subject's responses. This improves the progression of the subject. For example, if a lack of dexterity is detected in response to the measurements of a cognitive response of the subject, the last stimulus is repeated. Alternatively, or additionally, if a lack of dexterity is detected in response to the measurements of the kinematics of the subject, repeating the former stimulus. Alternatively, or additionally, if a lack of dexterity is detected in response to the measurements of desynchronization by using the EEG techniques, repeating the first stimulus of the set of stimuli. Here the expression lack of dexterity detected in response to the measure of a cognitive response of the subject and/or the measurements of the kinematics of the subject and/or the measure of desynchronization by using the EEG techniques means that one or more of these measurements have been obtained with a value that is above or below limits of normal values. The repetition of the stimulus causing the detection of lack of dexterity allows the subject to improve the responses to the repeated stimulus.

An example of implementation of predicting (estimating) the motor performance of the subject using time series data (S20) - from D1 to D5, including bloc and session data- by machine learning techniques. Steps involved in the classification of time series data are now outlined, including data preprocessing, feature extraction, model selection, and evaluation. Time

Data Collection and Pre-processing: raw time series data may contain errors and missing values, which need to be addressed. This is performed as known in the art.

Feature extraction: feature engineering techniques as known in the art are applied to transform raw data into meaningful input for classification models. Relevant features are extracted from the time series data to capture important patterns. These features include 1) time domain features and 2) frequency domain features.

1)The time domain features include the maximum velocity of finger movement, the smoothness of hand trajectory and the pic-to-pic angular displacement for the Mocap data.

2)For extracting frequency domain features, the Discrete Cosine Transform (DCT-III) is applied to each of the windowed segments independently. The DCT coefficients represent the signal's frequency components in the given window.

Components in the Frequency Domain Shows information about the frequency content of the signal are represented by the formula:

$$X_k = \sum_{n=0}^{N-1} x_n \cos\left[ \frac{\pi}{N} \left( n + \frac{1}{2} \right) k \right] \qquad \text{for } k = 0, \ldots N - 1.$$

Component 0 (DC Component) represents the average value or the "DC offset" of the signal. The first few components capture the low-frequency components of the signal (e.g., slow variations or trends). The higher-numbered components represent increasingly higher frequencies (represent higher-frequency details or oscillations).

Labeling: data is labeled with appropriate categories or classes. Labels can be assigned manually with ground truth information or generated using clustering algorithms based on observed patterns. Here, it is supposed that first sessions have label 0 that means they have the lowest score, and the last sessions have label 1 that means they have the highest score.

Model Selection: Various classification algorithms can be used for time series classification, such as decision trees, Linear Discriminant Analysis (LDA), random forests, support vector machines, or deep learning models like recurrent neural networks (RNNs) or convolutional neural networks (CNNs). The choice of the algorithm depends on the nature of the data and the classification problem. Preferably, Linear Discriminant Analysis (LDA) is used. LDA is a dimensionality reduction and classification technique used in machine learning and statistics. It is primarily employed for solving classification problems, but it also has applications in feature extraction and data visualization. LDA aims to maximize the between-class scatter while minimizing the within-class scatter in the reduced-dimensional space. By doing so, LDA enhances class separability, making it easier for classification algorithms to discriminate between different classes.

Model Training: The selected model is trained on the training dataset using the extracted features (Cognitive, Motor and EEG responses). Hyperparameters are tuned to optimize model performance on the validation set, typically through techniques like cross-validation.

Model Evaluation: Model performance is evaluated using appropriate metrics, including but not limited to accuracy, precision, recall, F1-score, and ROC-AUC (Compute Area Under the Receiver Operating Characteristic Curve), depending on the specific classification problem. Visualizations such as confusion matrices and ROC curves provide insights into model performance.

Model Interpretation: For interpretable models, feature importance analysis is conducted to understand which features are driving classification decisions.

Prediction using for example LDA: The normalized LDA output for each session (performance score) is used as time series data. This can be compared to subject learning curves with 3 standard responses as shown on FIG. 16 with curves 160, 162, 164:

- the first standard response corresponds to the equation $y(t)=mt+c$ (curve160), where $y(t)$ is the level of skill at a given time t, m is the slope of the line, representing the constant rate of learning, and c is the y-intercept, representing the initial level;
- the second standard response is a strong improvement at the beginning reaching rapidly a plateau (curve 162) that is represented by the equation $y(t)=A * (1 - \exp(-Bt))$, where A is the maximal value of the performance score, B is a parameter that controls the speed at which the curve reaches the plateau;
- the third standard response has an improvement mainly at the end of the time series. It is represented by the equation $y(t) = A * (1 - \exp(-Bt^C))$, where A is the maximal value of the performance score, where B is a parameter that controls the speed at which the curve reaches the plateau, and C is a parameter that control the shape of the curve.

A distance measure is then used to compute the distance between subject's curve and the three standard profiles (160, 162, 164) in order to characterize the subject's sensorimotor adaptation profile.

A decision trees algorithm is then used to predict the behavior of the subject based on the past week score. A tree-like model is created that splits the data into different groups based on subject's identified past behavior. The tree can be used to make predictions about the individual's future behavior based on the group they belong to (profile a, b or c).

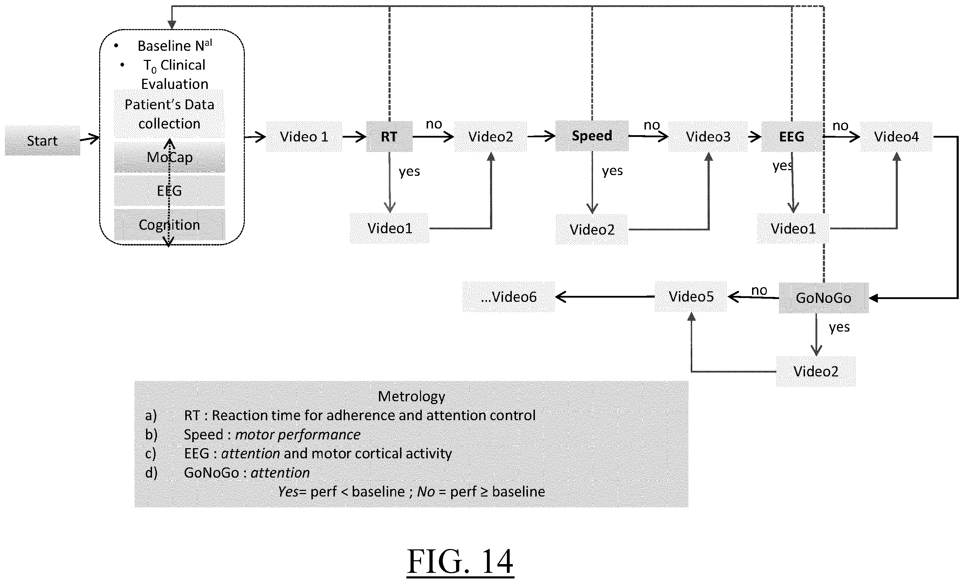

FIG. 14 is an example of decision tree of a supervised training of the subject, e.g., the neural networks adapt the training program online. A first stimulus is displayed to the subject (Video 1). From the short-time frame measurements, the adhesion of the subject is determined; this can be done using the collected measurements in response to the stimulus Video 1, e.g., reaction time is measured. If it is determined that the subject adheres to the stimulus, Video 1 is displayed again and then Video 2; if not, another video Video2 is displayed. Then, the execution time in response to the Video2 is measured; mainly using cognitive measures. If it is determined that the execution time of subject is good (e.g., by comparison with a standard subject), Video2 is displayed again and then Video3. If not, the Video3 is displayed. Attention and cortical changes are then scored using the collected responses of the subject to Video3, e.g., by measuring EEG. If the computed score indicated that the attention and cortical changes are good (e.g., still by comparison with a standard subject), Video 1 is displayed again; otherwise, Video 4 is displayed. Once Video 4 has been displayed, attention is measured and depending on the result, a new video 5 is display or a former video is shown to the user (e.g., Video 2). The training session (thus a short-time frame) will stop when two or more consecutive scores obtained from the measurement indicate that the subject is no more in a condition to improve motor cortical areas of their brain.

The present invention thus allows feeding a supervised learning program controlling the crucial recovery parameters such as task difficulty, number of repetitions and exercise duration. These variables are tuned considering the subject's performance throughout the training session. The learning model is bio-inspired and based on experimental evidence from neuroscience. Sensory-motor adaptation is based on the principle of homeostasis: a bad response of the subject to a stimulus of the training programme occurs when the balance is disrupted between the inputs (type of stimuli) and the quality of subjects' responses. Maintaining a balance between these two components is a control variable of the predictive model. Motivation and adherence are fundamental components of the recovery process. According to this architecture of supervised learning program, motivation depends on the ability to minimize prediction errors (the choice of stimuli proposed to the subject by the virtual assistant) on the value that is expected after a decision is made when the subject is faced with several alternatives.

**Claims**

1. A device for training motor cortical areas of a brain of a human subject, comprising:

>    - a display for providing the human subject with a set of visual stimuli;
>    - a speaker for providing the human subject with auditory stimuli;
>    - sensors for collecting responses of the human subject to the stimuli, the collecting comprising:
>
>>        -- collecting with a first sensor data for measuring a cognitive response of the human subject, the cognitive responses comprising the reaction time and/or error estimation of the subject in response to the stimulus and the duration of both explicit and implicit mental simulation of displayed body movements of the set of visual stimuli;
>>        -- measuring with a second sensor using electroencephalogram techniques, desynchronization of alpha waves of the brain, of low beta and Mu rhythm;
>>        -- measuring with a third sensor kinematics of at least one part of the body of the subject;
>
>    - from the collected responses, estimating by a processing unit a motor adaptation of the subject in response to one or more of the provided stimuli.

2. The device of claim 1, wherein the estimating the motor adaptation of the subject comprises:

>    - using a trained neural network to obtain an estimation of the motor adaptation of the subject, the neural network having been trained based on a dataset of collected responses of the subject to sets of visual and auditory stimuli, the neural network being configured for estimating a motor adaptation of the subject in response to one or more of the provided stimuli.

3. The device of claim 2, wherein the neural network having been further configured for estimating a dynamic of a training program of the motor cortical areas of the brain of the human subject along short and long-time frames.

4. The device of anyone of claims 1 to 3, wherein the dataset of collected responses and the sets of visual and auditory stimuli for the learning are obtained in a similar way as for the training.

5. The device of anyone of claims 1 to 4, wherein the collecting responses of the subject for the training is performed on the basis of:

>    - short-time frames, each short-time frame representing a subject training session of up to 30 minutes;
>    - long-time frames, each long-time frame being computed by consolidating the data of the short time frames on a weekly and/or monthly basis.

6. The device of anyone of claims 1 to 5, wherein the visual and/or auditory stimuli are produced using computer graphics tools and methods for activating a perceptual property of the brain, the perceptual property being selected among a motor resonance, an affordance, a visual inference allowing mental simulation of action.

7. The computer-implemented of anyone of claims 1 to 6, wherein the produced visual stimuli comprise images and/or videos with an impoverished appearance in structure and/or contour and/or color and/or speed and/or with an altered observer's point of view altered.

8. The device of anyone of claims 1 to 7, wherein measuring the reaction time comprises measuring a time lag between the actual end of a movement of the subject in response to the stimulus and an end of the movement estimated by the subject; and
wherein measuring an error estimation comprises:

>    - measuring an error in movement duration estimation of the displayed movement, and/or

- measuring an error in the subject's response to a visual stimulus when it must be associated with a sound stimulus and vice versa, an error occurring when an unexpected of response of the subject to the stimulus is performed.

9. The device of anyone of claims 1 to 8, wherein the measuring kinematics is performed with markerless techniques to extract kinematic biomarkers of the sensory-motor deficiency among which the maximum velocity, the time to peak velocity, the smoothness and the energy cost of the movement.

10. The device of anyone of claims 1 to 9, wherein:

- if a lack of dexterity is detected in response to the measurement of a cognitive response of the subject, repeating the last stimulus;
- if a lack of dexterity is detected in response to the measurement of the kinematics of the subject, repeating the former stimulus;
- if a lack of dexterity is detected in response to the measurement of desynchronization by using the EEG techniques, repeating the first stimulus of the set of stimuli.

11. The device method of anyone of claims 1 to 10, wherein the estimating motor adaptation of the subject in response to one or more of the provided stimuli comprises:

- comparing a sensory-motor adaptation of the subject in response to one or more of the provided stimuli with limits of normal values of the sensory-motor adaptation of the subject in response to one or more similar stimuli, the limits of normal values being obtained from a large group of non-affected subjects.

12. The device of any of claims 1 to 11, further comprising a memory communicatively coupled to the processing unit, the memory storing a computer program comprising instructions that when executed by the processing unit cause the device to estimate from the collected responses the motor adaptation of the subject in response to one or more of the provided stimuli.

13. The device of claim 12, wherein:

- the first sensor comprises a sound recorder;
- the second sensor comprises EEG electrodes;
- the third sensor comprises two motion capture cameras;

and wherein the computer program further comprises instructions, that when executed by the processing unit, cause the display to emit the set of visual stimuli, the speaker to emit auditory stimuli, and the sound record to record vocal responses of the subject.

14. A computer program comprising the instructions according to claim 12 and/or 13.

15. A computer readable medium storing the computer program of claim 14.

FIG. 1

The daily stimulation program is divided into 2 sessions (S1 in morning and S2 in the afternoon), each session consisting of 3 blocs of stimuli for a total of 30 minutes per session (one day training= 1h).

**Day *n***

| Session 1 Morning | | | S2 Afternoon | | | |
|---|---|---|---|---|---|---|
| B1 | B2 | B3 | B4 | B5 | B6 | Day n Scoring |

A full training program includes 4 weeks, each week consisting of 5 days, each day including 2 sessions, each session including 3 blocs.

**Week 1**

| S1 | S2 | S3 | S4 | S5 | S6 | S7 | S8 | S9 | S10 |
|---|---|---|---|---|---|---|---|---|---|
| Day1 | | D2 | | D3 | | D4 | | D5 | |

**4 Weeks training**

| D1 | D2 | D3 | D4 | D5 | D6 | ... | | | | | | | | | | | | | D20 |

# FIG. 2

**Bloc 1**

| Mocap1 | V1 | V2 | Cog1 | V3 | V4 | V5 | Cog2 | V6 | V7 | V8 | Cog3 | V9 | V10 | Mocap2 |

EEG Collection

Data base for 1 Bloc = 10 stimuli + 3 cognitives responses + 2 MoCap

| | Motor Resonance | Affordance | Inference | Mental Rotation | Planing | Attention |
|---|---|---|---|---|---|---|
| Stim visual | X | X | | | | |
| Stim audio | X | | | | | |
| Stim visual audio recognition | X | | | | | |
| Masking and time estimation | X | | X | | | X |
| Masking and spatial estimation | X | | X | | | X |
| Masking and object recognition | X | | X | | | X |
| Puzzle | X | X | | X | X | |
| Speed estimation | X | | | | | |
| Wheight estimation | X | X | | | | |
| Natural gesture estimation | X | X | | | | |
| Question about previous video | | | | | | X |
| Question about previous object | X | X | | | | X |
| Stroop Task | | | | | | X |
| GoNoGo task | | | | | | X |
| Reaction Time to simple sitmulus | | | | | | X |
| Action Pantomime recognition | X | X | | | | |
| Pantomime communication recognition | X | | | | | |
| Action selection from object | | X | | | | |
| Action recognition from point light dispaly (PLD) | X | | | | | |
| Video recognition from PLD | X | | | | | |
| Limb side recognition | | | | X | | |
| hand grip difficulty estimation | X | X | | X | | |

FIG. 3

FIG. 4

FIG. 5

Which object is the easiest to grasp ?

A          B          C

FIG. 6

Select the right Grip!

A          B          C

D          E          F

FIG. 7

FIG. 8

Select the sound that produced the previous seen action ?

A

B

C

FIG. 8a

**FIG. 9**

FIG. 10

FIG. 11

EP 4 552 575 A1

FIG. 12

EP 4 552 575 A1

Mocap Collection

Clinical evaluation t0 | Mocap1 | V1 | V2 | Cog1 | V3 | V4 | V5 | Cog2 | V6 | V7 | V8 | Cog3 | V9 | V10 | Mocap2

Motor Performance Evaluation

time(ms) 1120

SHOULDER

ELBOW

HAND

Elbow Angle

FIG. 13

EP 4 552 575 A1

FIG. 14

provide to the subject a set of visual and auditive stimuli — S10

for each stimulus, collect responses of the subject — S20

Collect data measuring a cognitive response of the subject — S22

measure, by using EEG techniques, desynchronization of alpha waves of the brain, of low beta and Mu rhythm — S24

measure kinematics of at least one part of the body of the subject in response to the stimulus — S26

from the collected responses, estimate a motor adaptation of the subject in response to one or more of the provided stimuli — S30

# FIG. 15

FIG. 16

Europäisches
Patentamt

European
Patent Office

Office européen
des brevets

# EUROPEAN SEARCH REPORT

**Application Number**

EP 23 30 6923

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BONI SARA ET AL: "Action Observation Therapy for Arm Recovery after Stroke: A Preliminary Investigation on a Novel Protocol with EEG Monitoring", JOURNAL OF CLINICAL MEDICINE, [Online] vol. 12, no. 4, 7 February 2023 (2023-02-07), page 1327, XP093116679, CH ISSN: 2077-0383, DOI: 10.3390/jcm12041327 [retrieved on 2024-01-11] * abstract * * Section 1, paragraph 2 * * Section 2.2, paragraph 1 and 2 * * Section 2.3, paragraph 1, 2 and 3 * * Section 2.4, paragraph 1 * * figure 2 * | 1-15 | INV. A61B5/375 A61B5/00 ADD. A61B5/16 G06F3/01 |
| A | WANG LEI ET AL: "Effects of rhythmic auditory stimulation on motor function and balance ability in stroke: A systematic review and meta-analysis of clinical randomized controlled studies", FRONTIERS IN NEUROSCIENCE, vol. 16, 17 November 2022 (2022-11-17), XP093117731, CH ISSN: 1662-453X, DOI: 10.3389/fnins.2022.1043575 [retrieved on 2024-01-11] * abstract * | 1-15 | TECHNICAL FIELDS SEARCHED (IPC) A61B G06F |

-/--

The present search report has been drawn up for all claims

| Place of search | Date of completion of the search | Examiner |
|---|---|---|
| The Hague | 22 January 2024 | Lai, Marco |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 3

Europäisches
Patentamt
European
Patent Office
Office européen
des brevets

# EUROPEAN SEARCH REPORT

Application Number

EP 23 30 6923

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BENNABI DJAMILA ET AL: "Motor Resonance Mechanisms during Action Imitation in Depression", NEUROPSYCHIATRY, [Online] vol. 08, no. 05, 1 January 2018 (2018-01-01), XP093117803, GB ISSN: 1758-2008, DOI: 10.4172/Neuropsychiatry.1000484 [retrieved on 2024-01-11] * abstract * * Section "Movement tasks"; page 1505 - page 1506; figure 1 * * Section "Movement tasks", Subsection "Movement observation experiment"; page 1505 - page 1506 * * Section "Data analysis"; page 1506 * | 1-15 | |
| A | CHOO YOO JIN ET AL: "Use of Machine Learning in Stroke Rehabilitation: A Narrative Review", BRAIN & NEUROREHABILITATION, [Online] vol. 15, no. 3, 31 October 2022 (2022-10-31), XP093118117, ISSN: 1976-8753, DOI: 10.12786/bn.2022.15.e26 [retrieved on 2024-01-11] * Abstract and highlights * | 1-15 | **TECHNICAL FIELDS SEARCHED (IPC)** |

-/--

The present search report has been drawn up for all claims

| Place of search | Date of completion of the search | Examiner |
|---|---|---|
| The Hague | 22 January 2024 | Lai, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 23 30 6923**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MATHIS ALEXANDER ET AL: "DeepLabCut: markerless pose estimation of user-defined body parts with deep learning", NATURE NEUROSCIENCE, NATURE PUBLISHING GROUP US, NEW YORK, vol. 21, no. 9, 20 August 2018 (2018-08-20), pages 1281-1289, XP036579324, ISSN: 1097-6256, DOI: 10.1038/S41593-018-0209-Y [retrieved on 2018-08-20] * abstract * | 1-15 | |
| A | MIKOS VAL ET AL: "Regression analysis of gait parameters and mobility measures in a healthy cohort for subject-specific normative values", PLOS ONE, vol. 13, no. 6, 18 January 2018 (2018-01-18), page e0199215, XP093118176, US ISSN: 1932-6203, DOI: 10.1371/journal.pone.0199215 * Introduction, paragraph 1 * | 1-15 | TECHNICAL FIELDS SEARCHED (IPC) |

The present search report has been drawn up for all claims

| Place of search | Date of completion of the search | Examiner |
|---|---|---|
| The Hague | 22 January 2024 | Lai, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 3 of 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *The mirror-neuron system, Annual Review of Neuroscience*, 21 July 2004, vol. 27, 169-192
- **GIBSON, J. J.** *An ecological approach to perception. Boston (MA): Houghton Mifflin*, 1979
- **POZZO et al.** Kinematic features of movement tunes perception and action coupling. *Behavioural Brain Research*, 25 April 2006, vol. 169 (1), 75-82
- **H. HOBSTON et al.** The interpretation of mu suppression as an index of mirror neuron activity: past, present and future. *Royal Society Open Science*, March 2017, vol. 4 (3)

- **S. BONI et al.** Action Observation Therapy for Arm Recovery after Stroke: A Preliminary Investigation on a Novel Protocol with EEG Monitoring. *J. Clin. Med.*, 2023, vol. 12 (4), 1327
- **A. MATHIS et al.** DeepLabCut: markerless pose estimation of user-defined body parts with deep learning. *Nature Neuroscience*, 2018, vol. 21, 1281-1289
- **B. BERRET et al.** *Evidence for Composite Cost Functions in Arm Movement Planning: An Inverse Optimal Control Approach*, 13 October 2011